# EUROPEAN PATENT APPLICATION

(11) **EP 1 285 662 A1**
(43) Date of publication of application: **26.02.2003**
(21) Application number: 01120026.8
(22) Date of filing: 20.08.2001
(51) Int. Cl.: A61K 38/17

(54) **Reconstituted HDL for the treatment of stroke and ischemic conditions**

(71) Applicant: ZLB Bioplasma AG, 3000 Bern 22 (CH)
(72) Inventor: Hubsch, Alphonse, 3052 Zollikofen (CH); Lang, Markus, 4105 Biel-Benken (CH); Lerch, Peter, 3007 Bern (CH)
(74) Representative: Weiss, Wolfgang, Dipl.-Chem. Dr.

(57) **Abstract**

The present invention relates to a method for the prophylaxis and/or treatment of stroke and ischemic conditions, wherein reconstituted HDL (rHDL) is administered to a subject in need thereof, particularly by intravenous infusion.

## Description

The present invention relates to a method for the prophylaxis and/or treatment of stroke and ischemic conditions, wherein HDL particles, preferably reconstituted HDL (rHDL) particles are administered to a subject in need thereof, particularly by intravenous infusion.

Stroke can be classified into thrombo-embolic and hemorrhagic forms and is the third largest cause of death in western countries, after heart disease and cancer. In the United States each year 600 000 people suffer a new or recurrent stroke (about 500 000 are the first attacks) and approximately 29% of them die within the first year (1). The incidence of stroke increases with age, and in the elderly it is the leading cause of serious, long-term disability in the US accounting for total costs of 51.3 billion $/year (1). Although the death rate from stroke has been decreasing in recent years, largely due to the increased awareness and better control of risk factors such as hypertension, hypercholesterolemia, arrhythmia or diabetes, the actual number of stroke deaths is rising because of an increasing elderly population. However, when prevention measures fail only limited and risky thrombolytic approaches exist, e.g. t-PA (tissue plasminogen activator). Neuronal protection could become a new and safer strategy for stroke treatment in the future (2-4).

One common cause of circulatory shock is severe blood loss associated with trauma. Despite improvements in intensive care medicine, mortality from from hemorrhagic shock remains high (5,6). Thus, there is still a great need for new approaches to improve therapy and outcome of patients with hemorrhagic shock (6). In clinical practice, hemorrhagic shock leads to a delayed vascular decompensation (resulting in severe hypotension) and, in approximately 25% of patients, in the dysfunction or failure of several organs including lung, kidney, gut, liver and brain (7). There is evidence that both ischemia (due to reduced blood and oxygen supply during hemorrhage) and reperfusion (during resuscitation) play an important role in the pathophysiology of the multiple organ dysfunction syndrome (MODS) in hemorrhagic shock (8).

Reconstituted high density lipoprotein (rHDL) may be prepared from human apolipoprotein A-I (apoA-I), e.g. isolated from human plasma, and soybean-derived phosphatidylcholine (PC), mixed in molar ratios of approximately 1:150 apoA-1:PC. The particles which are formed, are similar to nascent HDL. rHDL has been shown to stimulate cholesterol efflux from peripheral cells in a process better known as reverse cholesterol transport. Furthermore, rHDL dose-dependently binds LPS and inhibits LPS-induced cytokine production as well as adherence of PMNs (polymorphonuclear leukocytes) to endothelial cells. rHDL has anti-inflammatory and free oxygen radical scavenger activity. rHDL also decreases the rate and the extent of platelet aggregation. More recently it was demonstrated that rHDL acutely restores endothelial function and in turn normalizes blood flow in hypercholesterolemic patients as determined by forearm plethysmography (9). Thus, the pharmacological profile of rHDL implies a potential role for the compound in LPS-induced septicemia as well as in cardio- and cerebrovascular disease.

The pathophysiology of stroke is characterized by a wide-range of homeostatic, hemodynamic and metabolic abnormalities such as thrombus formation, impaired endothelial function and an activated inflammation cascade, i.e. increased cytokine production and expression of adhesion molecules (10-15). Another hallmark of stroke is the augmented oxidative stress after reperfusion which is thought to play a detrimental role in the progression of the disease.

Prolonged ischemia results in an elevation of intracellular Ca⁺⁺ and the consequent activation of proteases and phospholipases results in formation of numerous potentially damaging products of membrane lipid breakdown. These include arachiodonic acid metabolites, which, in the presence of oxygen during reperfusion, provide a source of free radical formation (e.g. superoxide and hydroxyl anions). These free radicals induce blood brain barrier destruction and neuronal apoptosis and/or necrosis. Apoptosis is a form of cell death that eliminates compromised or superfluous cells with no inflammatory response and is differentiated from necrosis by many morphological and biochemical characteristics. The feature of apoptosis can be found in both neurons and glia after ischemic injuries. Neurons in the ischemic penumbra, that are not exposed to lethal ischemia, may undergo delayed apoptosis (16). The so called penumbra is a brain area where blood flow is reduced to a level that interrupts neuronal function and the consequent electrical activities, yet permits maintenance of membrane pumps and preservations of ion gradients. This brain area has two characteristics that explain its potential clinical importance: 1) the interruption of clinical and electrical function that characterizes this area is fundamentally reversible, but 2) the reversibility is time-limited and linked to reperfusion.

Surprisingly, it was found that the size of the lesions in animal models for stroke (exocitotoxicity and cerebral artery occlusions) is reduced by administration of rHDL. These data show that rHDL can improve the outcome following exocitotoxic and ischemic/reperfusion neuronal damage, particulary apoptosis and/or necrosis in the ischemic area and in the penumbra. Further, it was shown in an animal model for hemorrhagic shock that rHDL reduces the PMN infiltration and prevents organ injury and dysfunction. At present, the mechanism of action is unknown. While not wishing to be bound by theory, it is possible that rHDL might act as free oxygen radical scavanger, vasodilator, e.g. via improvement of NO bioavailability resulting in an improvement of collateral blood flow or exhibits an anti-inflammatory effect. Thus, rHDL may act as a neuroprotective drug particularly in cerebrovascular diseases.

Thus, a subject matter of the present invention is the use of HDL for the manufacture of an agent for the prophylaxis and/or treatment of ischemia or reperfusion injury. Particularly, HDL may be used for the prophylaxis and/or treatment of a disorder selected from ischemic stroke, cardiac ischemia, cardiac reperfusion injury, resuscitation from hemorrhagic shock, and complications resulting from organ transplantation, e.g. kidney, heart, liver, etc. or cardio-pulmonary bypass surgery.

The term "HDL" as used in the present invention relates to particles similar to high density lipoproteins and comprises nascent HDL or reconstituted HDL (rHDL) or any mixture thereof. Such particles can be produced from a protein or peptide component, and from lipids.

The proteins are preferably apolipoproteins, e.g. human apolipoproteins or recombinant apolipoproteins, or peptides with similar properties. Suitable lipids are phospholipids, preferably phosphatidyl choline, optionally mixed with other lipids (cholesterol, cholesterol esters, triglycerides, or other lipids).

Administration of HDL may result, on one hand, in a short term effect, i.e. an immediate beneficial effect on several clinical parameters is observed, and, on the other hand, a long term effect, a beneficial alteration on the lipid profile may be obtained. Furthermore, HDL resembles very closely substances naturally occuring in the body and thus the administration of HDL should be substantially free of side effects.

HDL is preferably administered by infusion, e.g. by arterial, intraperitoneal or preferably intravenous injection and/or infusion in a dosage which is sufficient to obtain the desired pharmacological effect. For example, HDL may be administered before the start of ischemia (if foreseeable, e.g. before an organ transplantation) and/or during ischemia, before and/or shortly after reperfusion, particularly within 24 h-48 h.

The HDL dosage ranges preferably from 10-200 mg, more preferably 40-80 mg HDL (weight based on apolipoprotein) per kg per treatment. For example, the dosage of HDL which is administered may be about 20-100 mg HDL per kg (weight based on apolipoprotein) given as a bolus injection and/or as an infusion for a period ranging from a few minutes to several hours. If necessary, the HDL administration may be repeated one or several times.

According to the present invention, an HDL, e.g. nascent HDL, rHDL or HDL-like particle is particularly preferred which has a molar ratio of protein, e.g. apolipoprotein A-I and phospholipid in the range of 1:50 to 1:250, particularly about 1:150. Further, rHDL may optionally contain additional lipids such as cholesterol, cholesterol esters, triglycerides and/or sphingolipids, preferably in a molar ratio of up to 1:20, e.g. 1:5 to 1:20 based on the apolipoprotein.

The administration of HDL may be combined with the administration of other pharmaceutical agents such as thrombolytic agents, antiinflammatory agents, neuro- and/or cardioprotective agents.

Furthermore, the present invention relates to a method for prophylaxis and/or treatment of ischemia or reperfusion injury comprising administering a subject in need thereof an effective amount of HDL. Preferably, HDL is administered to a human patient.

Further, the present invention shall be explained in detail by the following examples:

### Example 1

### 1. General Methods

Exocitotoxic lesion: Experiments were performed in Sprague-Dawley rats anesthetized with chloral hydrate (400 mg/kg ip). A femoral vein was cannulated for infusion of rHDL. Rats were placed into a stereotaxic apparatus and, after a midline incision, received a unilateral injection of N-methyl-D-aspartate (NMDA) or vehicle into the right striatum: coordinates: 0.2 mm posterior, 3 mm lateral, 5.5 mm ventral to the bregma. Five minutes after insertion of the needle the solution was injected over a period of 6 minutes using a Hamilton syringe pump at a rate of 0.5 ml/min. 5 minutes after injection has been completed, the needle was removed.

Middle cerebral artery occlusion: Experiments were performed in Sprague-Dawley rats anesthesized with chloral hydrate (400 mg/kg ip). The trachea were cannulated and the animals were mechanically ventilated with air and supplemental oxygen to maintain blood gases within normal ranges. Rectal temperature was continually monitored and maintained at 37°C. Catheters were placed into the femoral artery to measure systemic blood pressure and to monitor blood gases. A femoral vein was cannulated for infusion of drug. A neck midline incision was made and the right common carotid artery was exposed. Following coagulation of its branches, the external carotid artery (ECA) was distally opened. A nylon thread (diameter 0.22 mm) which has a distal cylinder of silicon (2 mm long, diameter 0.38 mm) of thermofusible glue was inserted in the lumen of ECA and advanced into the internal carotid artery up the origin of MCA. To restore the MCA blood flow, the nylon thread was removed and cut thirty minutes later.

Histological analysis: Twenty-four hours after the surgery euthanasia was performed. The brains were rapidly removed, frozen in isopentane at -50°C and stored at -80°C. Cryostat cut coronal brain sections (20 µm) were stained with thionine and analyzed using an image analyzer. The lesioned areas were delimited by the paleness of histological staining in alterated tissue compared to the color of healthy tissue. Regions of interest were determined through the use of a stereotaxic atlas for the rat and an image analysis system was used to measure the lesioned area.

### 2. Experimental Protocols

### Exocitotoxic lesion

In this series of experiments rats received intravenous infusion of saline (n = 5) (5 µl/min) over 4 h. After 2 h, unilateral injection of NMDA (75 nM in 3 ml of phosphate-buffered saline pH 7.4) was performed into the right striatum. After twenty-four hours, rats were sacrificed and the brain was removed for histological analysis. In another group of experiments, rats received intravenous infusion of rHDL (n = 5) (5 µl/min) at a dose of 120 mg/kg over 4 h. After 2 h, unilateral injection of NMDA (75 nM in 3 ml of phosphate-buffered saline pH 7.4) was applied into the right striatum and intravenous infusion of rHDL continued for additional 2 h. Twenty-four hours later the rats were sacrificed and the brain was removed for histological analysis.

### Middle cerebral artery (MCA) occlusion

In this series of experiments rats received an intravenous infusion of saline (n = 5) (5 µl/min) over 4 h. After 2 h the MCA of rats was occcluded for 30 minutes followed by reperfusion. After twenty-four hours, rats were sacrified for histological analysis of the brain. In another group of experiments, rats received intravenous infusion of rHDL (n = 5) (5 µl/min) at a dose of 120 mg/kg over 4 h. After 2 h the MCA of rats were occluded for 30 minutes followed by reperfusion. Twenty-four hours later the rats were sacrificed for histological analysis of the brain.

In the exocitotoxic lesion model the following results were obtained:

**Table 1:**

| lesion volume in mm³ | | |
|---|---|---|
| rat | control | rHDL |
| 1 | 50.27 | 16.54 |
| 2 | 47.05 | 18.86 |
| 3 | 41.28 | 17.44 |
| 4 | 38.5 | 17.51 |
| 5 | 51.66 | 19.86 |
| n | 5 | 5 |
| MEAN | 45.75 | 18.04 |
| SD | 5.69 | 1.31 |
| SEM | 2.55 | 0.59 |

In these experiments a dramatic reduction of the brain necrotic volume in rHDL treated animals by 60.6% compared to controls was observed.

In the MCA occlusion model, the following results were obtained:

**Table 2:**

| Lesion volume in mm³ | | |
|---|---|---|
| rat | control | rHDL |
| 1 | 158.94 | 54.18 |
| 2 | 229.78 | 35.27 |
| 3 | 201.52 | 37.64 |
| 4 | 193.02 | 34.64 |
| 5 | 210.24 | 76.74 |
| n | 5.00 | 5.00 |
| MEAN | 198.70 | 47.69 |
| SD | 26.08 | 18.11 |
| SEM | 11.66 | 8.10 |

rHDL reduced brain necrotic volume by 76% as compared to control rats.

Conclusion: In both models, a dramatic reduction of the infarct volume was seen in rHDL treated animals, as compared to placebo treated controls: Exocitotoxic model: 60.6% reduction of necrotic volume; MCA occlusion model: 76% reduction.

### Example 2

Male Wistar rats were anesthesized with thiopentone. Catheters were implanted in the right femoral artery for the measurement of blood pressure, the right carotid artery to bleed the animals, and the jugular vein for the administration of drugs. The bladder was canulated to facilitate urine flow and to prevent post-renal failure. Upon completion of the surgical procedure, cardiovascular parameters were allowed to stabilize for 15 min.

Then blood was withdrawn from the carotid artery catheter in order to achieve a fall in mean arterial pressure (MAP) to 50 mm Hg within 10 min. Thereafter, MAP was maintained at 50 mm Hg for 90 min by either withdrawal or re-injection of blood. At 90 min after initiation of hemorrhage, the animals were resuscitated by re-injection of the remaining shed blood. At the same time, an i.v. infusion of isotonic saline (1.5 ml/kg/h) was started as fluid replacement and maintained throughout the experiment. The following groups were studied:
1. Sham control group: Rats were subjected to the surgical procedure without causing hemorrhage and treated with saline (1 ml/kg, i.v. bolus followed by an i.v. infusion of 1.5 ml/kg/h. n=9).
2. Sham rHDL control group: Rats were subjected to the same surgical procedure without causing hemorrhage, but received rHDL (80 mg apoA-l/kg i.v. bolus followed by an infusion of 1.5 ml/kg/h. n=5).
3. Hemorrhage control group: One minute prior to resuscitation with shed blood, the rats were treated with saline (1 ml/kg, i.v. bolus followed by an i.v. infusion of 1.5 ml/kg/h. n=7).
4. Hemorrhage rHDL group: At 1 min prior to resuscitation, animals received rHDL (80 mg apoA-l/kg i.v. bolus followed by an infusion of 1.5 ml/kg/h. n=10)

The animals were observed for 4 h after resusciation, then sacrificed. Blood samples were taken at baseline and 4 h after resuscitation and the following markers of tissue dysfunction were measured in serum: Liver injury was assessed by measuring alanine aminotransferase (ALT) and aspartate aminotransferase (AST), renal dysfunction was assessed by measuring plasma levels of creatinine and urea, serum total lipase was determined as an indicator of pancreatic injury and total creatine kinase (CK) activity was measured as an indicator of neural and/or muscular injury. Tissue samples from lung, kidney and small intestine were taken and processed for histopatholical analysis. Myeloperoxidase activity (MPO), an indicator of PMN accumulation and malondialdehyde, an indicator of lipid peroxidation were determined in samples of lung and kidney.

### Results:

In the hemorrhage control rats, resuscitation with shed blood led to an immediate increase in blood pressure from approximately 50 mm Hg to 113 ± 4 mm Hg. Thereafter, there was a progressive slow decline in MAP to approximately 65 mm Hg by the end of the experiment. Administration of rHDL had no significant effect on this hemodynamic response to hemorrhage and resuscitation. In the hemorrhage control group, hemorrhage and resusciation led to an increase in serum urea, creatinine, AST, ALT, lipase and CK, as compared to the sham control group. Treatment with rHDL significantly attenuated all of these effects. Histologically, lung, small intestine and kidney from rats subjected to hemorrhage and resuscitation showed edema and loss of normal tissue structure. In contrast, organs from animals which had received rHDL prior to resuscitation did not show these changes in morphology. In addition, there was less leukocyte infiltration in rHDL treated rats than in the hemorrhage control group. PMN infiltration in the lungs and kidney of rats subjected to hemorrhage and resuscitations was confirmed by increased tissue MPO levels, also this effect was attenuated by treatment with rHDL. Rats from the hemorrhage control group showed increased MDA levels in lungs and kidneys; this increase in MDA was attenuated by treatment with rHDL.

### Conclusion:

In a model of hemorrhagic shock, the administration of rHDL prior to resuscitation reduced PMN infiltration and prevented organ injury and dysfunction.

### Literature:

1. American Heart Association (AHA), 2000
2. Hays SJ. Therapeutic approaches to the treatment of neuroinflammatory diseases. Curr Pharm Des 4:335-348, 1998
3. Jean WC, Spellman SR, Nussbaum ES, Low WC, Reperfusion injury after focal cerebral ischemia: the role of inflammation and the therapeutic horizon. Neurosurgery 43:1382-1396, 1998
4. Barone FC, Feuerstein GZ. Inflammatory mediators and stroke: new opportunities for novel therapeutics. J Cereb Blood Flow Metab 19:819-834, 1999
5. Morgan WM, and O'Neill JA. Hemorrhagic and obstructive shock in pediatric patients. New Horiz 6: 150-154, 1998
6. Demetriades D, Smith JS, Jacobson LE, Moncure M, Minei J, Nelson BJ and Scannon PJ. Bactericidal/permeability-increased protein (rBP121) in patients with hemorrhage due to trauma: results of a multicancer phase II clinical trial. RBP121 Acute Hemorrhage Trauma Study Group. J Trauma 46: 667-676, 1999
7. Regel G, Gotz M, Weltner T, Sturm JA and Tscherne H. Pattern of organ failure following severe trauma. World J Surg 20: 422-429, 1996
8. Cryer HG. Therapeutic approaches for clinical ischaemia and reperfusion injury. Shock 8: 26-32, 1997
9. Spieker LE, Sudano I, Lerch PG, Lang MG, Binggeli C, Corti R, Lüscher TF, Noll G. High-density lipoprotein restores endothelial function in hypercholesterolemic men. N EngI J Med, 2000. (in preparation)
10. Feuerstein GZ, Wang X, Barone FC. The role of cytokines in the neuropathology of stroke and neurotrauma. Neuroimmunomodulation 5:143-159, 1998
11. DeGraba TJ. The role of inflammation after acute stroke: utility of pursuing anti-adhesion molecule therapy. Neurology 51:62-68, 1998
12. Benveniste EN. Cytokine actions in the central nervous system. Cytokine Growth Factor Rev 9:259-275, 1998
13. Van Wagoner NJ, Benveniste EN. Interleukin-6 expression and regulation in astrocytes. J. Neuroimmunol 100:124-139, 1999
14. Touzani O, Boutin H, Chuquet J, Rothwell N. Potential mechanism of interleukin-1 involvement in cerebral ischemia. J Neuroimmunol 100:203-215, 1999
15. del Zoppo G, Ginis I, Hallenbeck JM, ladecola C, Wang X, Feuerstein GZ. Inflammation and stroke: putative role for cytokines, adhesion molecules and iNOS in brain responses to ischemia. Brain Pathol 10:95-112, 2000
16. Du C, R Hu, CA Csernansky, CY Hsu, DW Choi. Very delayed infarction after mild focal cerebral ischemia: A role for apoptosis? J Cereb Blood Flow Metab 16:195-201, 1996
17. Matsuda Y, Hirata K, Inoue N, Suematsu M, Kawahima S, Akita H, Yokoyama M. High density lipoprotein reverses inhibitory effect of oxidized low density lipoprotein on endothelium-dependent arterial relaxation. Circ Res 72(5):1103-1109, 1993
18. Chander R, Kapoor NK. High density lipoprotein is a scavanger of superoxide anions. Biochem Pharmacol 40(7):1663-1665, 1990
19. Araujo FB, Barbosa DS, Hsin CY, Maranhao RC, Abdalla DS. Evaluation of oxidative stress in patients with hyperlipidemia. Atherosclerosis 117(1):61-71, 1995
20. Huang JM, Huang ZX, Zhu W. Mechanism of high-density lipoprotein subfractions inhibiting copper-catalyzed oxidation of low-density lipoprotein. Clin Biochem 31(7):537-543, 1998

## Claims

1. Use of HDL for the manufacture of an agent for the prophylaxis and/or treatment of ischemia or reperfusion injury.

2. The use of claim 1 for the prophylaxis and/or treatment of a disorder selected from ischemic stroke, cardiac ischemia, cardiac reperfusion injury, hemorrhagic shock and complications resulting from organ transplantation or cardio-pulmonary bypass surgery.

3. The use of claim 1 or 2 wherein HDL is administered by intravenous infusion and/or injection.

4. The use of any one of claims 1-3 wherein HDL is administered before the start of ischemia and/or during ischemia.

5. The use of any one of claims 1-3 wherein HDL is administered at or after reperfusion.

6. The use of any one of claims 1-5 wherein HDL is administered in a dosage of from 10-200 mg HDL (weight based on apolipoprotein) per kg per treatment.

7. The use of any one of claims 1-6 wherein HDL is administered as a bolus injection and/or as an infusion for a period of from a few minutes to several hours.

8. The use of any one of claims 1-7 wherein the HDL has a molar ratio of protein, e.g. apolipoprotein A-I to phospholipids in the range of 1:50-1:250 and optionally additional lipids such as cholesterol, cholesterol esters, trigylcerides and/or sphingolipids are present in a molar ratio of up to 1:20 based on the protein.

9. The use of any one of claims 1-8, wherein the HDL is selected from nascent HDL, rHDL or mixtures thereof.

10. The use of any one of claims 1-9 wherein HDL is administered in combination with other pharmaceutical agents.

11. A method for prophylaxis and/or treatment of ischemia or reperfusion injury comprising administering a subject in need thereof an effective amount of HDL.

12. The method of claim 11, wherein the HDL is selected from nascent HDL, rHDL or mixtures thereof.

13. The method of claim 11 or 12 wherein the subject is a human.
